(19) Europäisches Patentamt
European Patent Office
Office européen des brevets

(11) Numéro de publication: **0 198 762**
**B1**

(12) # FASCICULE DE BREVET EUROPEEN

(45) Date de publication du fascicule du brevet: **26.10.88**

(51) Int. Cl.⁴: **C 07 D 313/12,** A 61 K 31/335

(21) Numéro de dépôt: **86400709.1**

(22) Date de dépôt: **02.04.86**

(54) **Dérivés d'acide dibenzo[be]oxépinne-acétique, leur préparation, et des médicaments les contenant.**

(30) Priorité: **11.04.85 FR 8505425**

(43) Date de publication de la demande: **22.10.86 Bulletin 86/43**

(45) Mention de la délivrance du brevet: **26.10.88 Bulletin 88/43**

(84) Etats contractants désignés: **AT BE CH DE FR GB IT LI LU NL SE**

(56) Documents cités:
**EP - A - 0 037 254**
**FR - A - 2 242 976**

(73) Titulaire: **SYNTHELABO, 58, rue de la Glacière, F-75621 Paris Cedex 13 (FR)**

(72) Inventeur: **Purcell, Thomas, 47 bis, rue de la Millière Les Mesnils, F-78490 Montfort L'Amaury (FR)**
Inventeur: **Zard, Lydia, 6, impasse des Quatre Vents La Croix Audierne, F-91190 Gif S/Yvette (FR)**

(74) Mandataire: **Ludwig, Jacques et al, SYNTHELABO Service Brevets 58, rue de la Glacière, F-75621 Paris Cedex 13 (FR)**



Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention a pour objet des dérivés d'acide dibenzo[be]oxépinne-acétique, leur préparation et leur application en thérapeutique.

Les composés de l'invention répondent à la formule générale I,

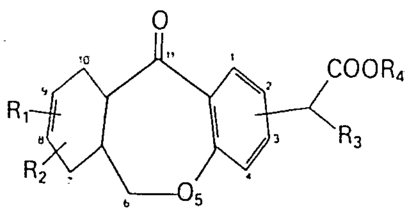

(I)

dans laquelle $R_1$, pris isolément, représente un atome d'hydrogène ou un groupe alkyle en $C_1$–$C_4$, et $R_2$, pris isolément, représente un atome d'hydrogène, un groupe alkyle en $C_1$–$C_4$ ou un groupe phénylthio ou phénylsulfonyle, ou bien $R_1$ et $R_2$ forment ensemble un pont éthano ou, avec les deux atomes de carbone 8 et 9, forment ensemble un noyau benzo condensé, $R_3$ représente un atome d'hydrogène ou un groupe méthyle, et $R_4$ représente un atome d'hydrogène, un groupe alkyle en $C_1$–$C_4$, ou un cation d'une base acceptable en pharmacologie, le groupe CH ($R_3$) $COOR_4$ étant en position 2 ou 3.

Les diverses formes isomères que peuvent présenter les composés de formule I font également partie de l'invention. Les composés peuvent en effet présenter une isomérie cis ou trans par rapport à l'axe défini par les atomes de carbone 6a et 10a. Par ailleurs, lorsque $R_3$ représente un groupe méthyle, l'atome de carbone qui le porte est chiral, de même que les atomes 7 et 10 lorsqu'ils portent un groupe méthyle.

Parmi les composés de l'invention, on préfère ceux dans la formule desquels $R_3$ et $R_4$ représentent chacun un atome d'hydrogène, et plus particulièrement ceux dans la formule desquels $R_1$ et $R_2$ représentent chacun, simultanément, un atome d'hydrogène ou un groupe méthyle, liés de préférence aux positions 8 et 9.

Des composés de structure analogue à celle des composés de l'invention et, comme ces derniers, présentant des activités anti-inflammatoires et analgésiques, sont décrits dans la demande de brevet européen n° 0 037 254 et dans la demande de brevet français n° 2 242 976. Toutefois, dans la structure des composés décrits dans le premier document, le cycle hexagonal condensé aux positions 6a–10a est entièrement saturé et, dans la structure des composés décrits dans le second document, le cycle correspondant est aromatique. En outre, une activité comme anti-agrégants plaquettaires de ces composés analogues connus n'est pas décrite dans les demandes de brevet susmentionnées. Conformément à l'invention, on peut préparer les composés de formule I selon le schéma donné à la page suivante.

On fait réagir d'abord un ester halogéné de formule II, dans laquelle R' représente un groupe alkyle en $C_1$–$C_4$, avec un hydroxyphénylacétate de formule III, dans laquelle R" représente un groupe alkyle en $C_1$–$C_4$ et $R_3$ est tel que défini ci-dessus, puis on hydrolyse le diester obtenu, de formule IV, en diacide de formule V. Ensuite on cyclise ce dernier de manière classique, par exemple en présence de chlorure de thionyle, pour obtenir le dérivé benzo [b] oxépinne-2-acétique de formule VI. On protège ce dernier en l'estérifiant (formule VII) avec un alcanol de formule ROH, R étant un alkyle en $C_1$–$C_4$ puis, en passant par l'intermédiaire 0-triméthylsilylé, on effectue une déshydrogénation donnant l'ester de formule VIII. On soumet enfin ce dernier à une réaction de Diels-Alder avec un butadiène de formule IX, dans laquelle $R_1$ et $R_2$ sont tels que définis ci-dessus, obtenant ainsi un ester de formule I ($R_4$ étant un groupe alkyle en $C_1$–$C_4$) que l'on peut hydrolyser pour obtenir un acide de formule I ($R_4$ étant un atome d'hydrogène). Lorsque l'on désire préparer des composés de formule I dans laquelle $R_3$ représente un groupe méthyle, on peut également partir d'un dérivé acétique de formule III, dans laquelle $R_3$ représente l'hydrogène, et insérer dans le procédé une étape de méthylation en α, de préférence une méthylation de l'ester de formule VII, avant la réaction de Diels-Alder.

Enfin, si l'on veut séparer les énantiomères, on peut par exemple faire réagir un acide de formule I ($R_4$=H) avec un énantiomère d'une base optiquement active, séparer les sels énantiomères ainsi obtenus par cristallisation fractionnée, et libérer les acides des deux fractions séparées.

Schéma

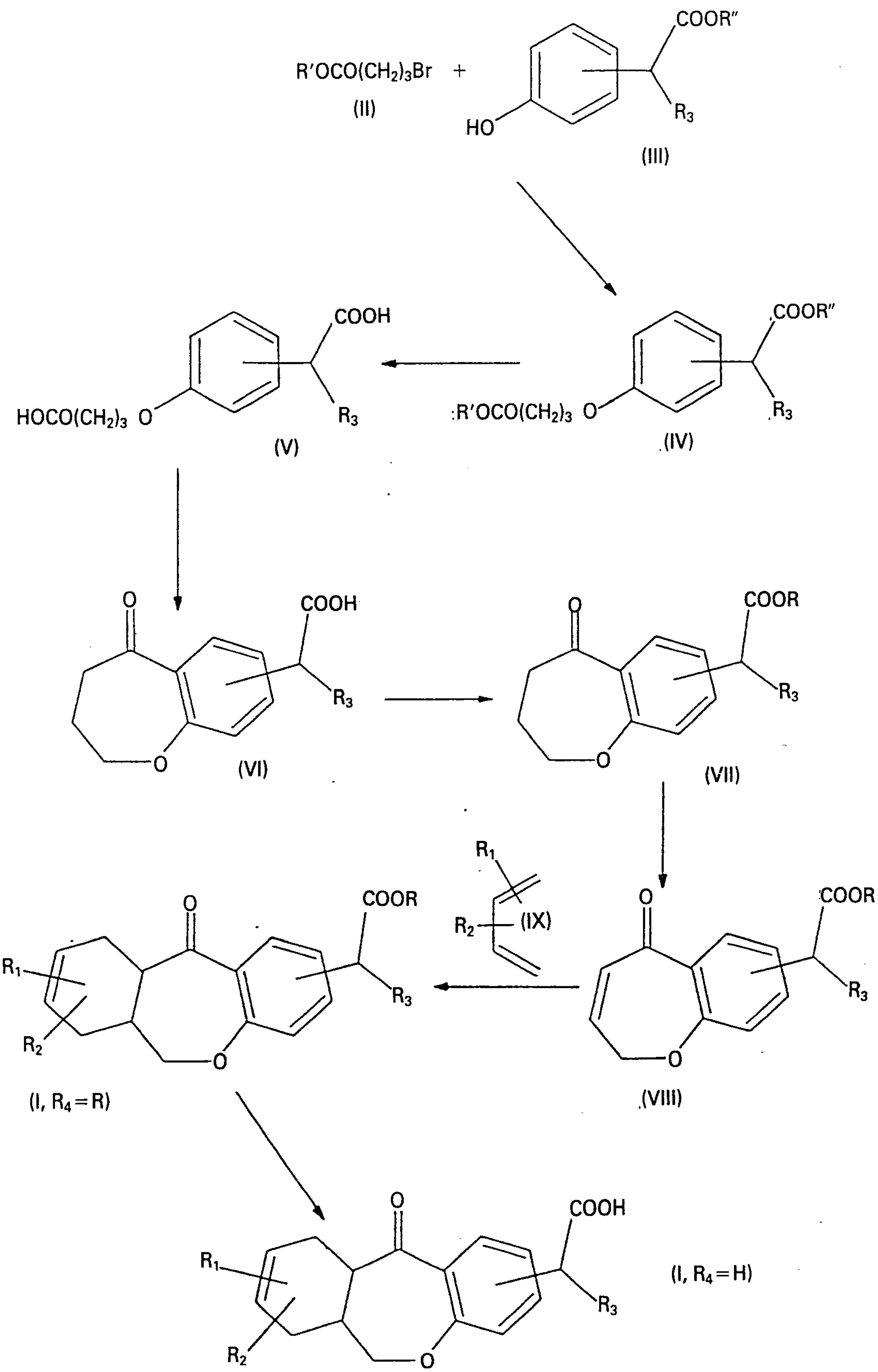

(II)

(III)

(IV)

(V)

(VI)

(VII)

(IX)

(VIII)

(I, $R_4 = R$)

(I, $R_4 = H$)

Les exemples suivants illustrent de manière détaillée la préparation de quelques composés selon l'invention. Les microanalyses et les spectres IR et RMN confirment les structures des composés obtenus.

Exemple 1. Acide oxo-11 hexahydro-6, 6a, 7, 10, 10a, 11 dibenzo[be]oxépinne-2-acétique.

a) (Hydroxy-4 phényl) acétate d'éthyle. On chauffe au reflux un mélange de 400 g d'acide (hydroxy-4 phényl) acétique, 2,5 l d'éthanol et 30 ml d'acide sulfurique à 96% pendant 5 h. On évapore à sec, on reprend le résidu avec 1 l de dichlorométhane, on lave avec une solution saturée de bicarbonate de sodium puis avec de l'eau. On sèche la phase organique et on l'évapore. Il reste une huile.

b) (Ethoxycarbonylméthyl-4 phénoxy)-4 butanoate d'éthyle. On fait réagir 45 g de sodium avec 1,6 l d'éthanol. On ajoute 279 g de l'ester précédemment préparé, puis 270 ml de bromo-4 butanoate d'éthyle et on chauffe le mélange au reflux pendant 10 h. On sépare le bromure de sodium par filtration, on évapore le filtrat, on le lave à l'eau et on le reprend avec du dichlorométhane. On lave la phase organique avec de la soude, puis avec de l'eau, on la sèche, on la filtre et on l'évapore. Il reste une huile.

c) Acide (carboxyméthyl-4 phénoxy)-4 butanoïque. On chauffe au reflux un mélange de 640 g de l'huile précédemment obtenue avec 624 ml de soude à 30% dans 1,3 l d'eau pendant 6 h. On acidifie le mélange obtenu, on sépare le précipité beige par filtration, on le lave à l'éther de pétrole et on le sèche.

d) Acide oxo-5 tétrahydro-2, 3, 4, 5 benzo [b]oxépinne-7-acétique. On chauffe à 90°C un mélange de 50 g du diacide précédemment préparé et de 500 g d'acide polyphosphorique pendant 3/4 h. On verse le mélange sur de l'eau glacée, on l'alcalinise avec de la soude à 33% jusqu'à pH 10, on le chauffe, on acidifie la phase aqueuse jusqu'à pH 3 et on l'extrait avec du dichlorométhane. On réunit les phases organiques, on les sèche, filtre et évapore le solvant. Il reste une huile.

e) Oxo-5 tétrahydro-2, 3, 4, 5 benzo [b]oxépinne-7-acétate de méthyle. On chauffe au reflux un mélange de 33 g de l'acide précédent, 200 ml de méthanol et 3 ml d'acide sulfurique pendant 4 h. On évapore le solvant et on distille l'huile résiduelle. On recueille une huile transparente.

f) Oxo-5 dihydro-2,5 benzo[b]oxépinne-7-acétate de méthyle. A un mélange de 60 g de l'ester précédent, 500 ml de dichlorométhane et 145 ml de triéthylamine on ajoute 40 ml de iodotriméthylsilane, tout en maintenant la température à moins de 20°C, sous atmosphère d'argon, et on laisse reposer le mélange à température ambiante. On chasse le solvant, on reprend le résidu dans de l'éther, on filtre les sels insolubles et on évapore l'éther. A une suspension de 77 g de dichlorodicyanoquinone dans 1 l de benzène on ajoute 15 ml de hexaméthyldisilazane puis, en une fois, le résidu d'évaporation précédent, et on agite le mélange pendant 2 h. On évapore le solvant et on chromatographie le résidu sur silice en éluant avec un mélange 20/80 d'acétate d'éthyle/cyclohexane. On recueille une huile rousse.

g) Oxo-11 hexahydro-6, 6a, 7, 10, 10a, 11 dibenzo[be]oxépinne-2 acétate de méthyle. On chauffe à 170°C un mélange de 2,5 g de l'huile précédente et 1,8 g de butadiène dans 15 ml de toluène pendant 40 h. On chasse le solvant et on chromatographie le résidu sur silice en éluant avec un mélange 90/10 de cyclohexane/acétate d'éthyle.

h) Acide oxo-11 hexahydro-6, 6a, 7, 10, 10a, 11 dibenzo[be]oxépinne-2-acétique. On hydrolyse l'ester précédent en en chauffant 1,5 g avec 0,63 g de soude à 30% à 50°C dans 17 ml d'eau et 17 ml de méthanol, de manière classique. On obtient finalement une huile qu'on laisse cristalliser dans l'éther de pétrole. Point de fusion: 112–113°C.

Exemple 2. Acide diméthyl-8,9 oxo-11 hexadydro-6, 6a, 7, 10, 10a, 11 dibenzo[be]oxépinne-3-acétique.

a) Acide oxo-5 tétrahydro-2, 3, 4, 5 benzo[b]oxépinne-8-acétique. En partant d'acide (hydroxy-3 phényl) acétique, et en manipulant comme dans les étapes 1a et 1c ci-dessus on obtient l'acide (carboxyméthyl-3 phénoxy)-4 butanoïque sous forme d'huile. On en introduit 50 g dans 500 ml de benzène, avec 60 ml de chlorure de thionyle et une trace de diméthylformamide, et on chauffe pendant 2 h au reflux. On chasse le solvant et on recueille une huile. On dissout cette dernière dans 400 ml de dichlorométhane et on l'ajoute goutte à goutte à une suspension de 37 g de chlorure d'aluminium dans 1 l de dichloroéthane, en maintenant la température entre −5 et +5°C. On agite 15 minutes à 0°C, puis on hydrolyse le mélange sur de la glace, on l'acidifie et on l'extrait au dichlorométhane. Après un traitement classique on recueille une huile.

b) Acide diméthyl-8,9 oxo-11 hexahydro -6, 6a, 7, 10, 10a, 11 dibenzo[be]oxépinne-3-acétique. En opérant comme aux exemples 1e à 1h, en effectuant toutefois la réaction de Diels-Alder avec le diméthyl-2,3 butadiène, on obtient un composé qui fond à 168–170°C.

c) Enantiomères de l'acide diméthyl-8,9 oxo-11 hexahydro −6, 6a, 7, 10, 10a, 11 dibenzo[be]oxépinne-3-acétique. On dissout 57,65 g (0,19 mole) du racémate précédemment obtenu et 61,6 g (0,19 mole) de quinidine dans 800 ml d'acétonitrile et on chauffe la solution au reflux. On arrête le chauffage et on ajoute 30 ml d'eau. Quelques cristaux apparaissent rapidement, puis le précipité se prend en masse. Après complet refroidissement on sépare la liqueur mère par filtration, on la conserve et on sèche les cristaux isolés. On les fait recristalliser dans un mélange de 450 ml d'acétonitrile et 50 ml d'eau, en chauffant au reflux puis en laissant refroidir très lentement. Après filtration et séchage du précipité on libère l'acide en traitant les cristaux avec de l'acide chlorhydrique à 10%, en ajoutant un minimum de dichlorométhane pour obtenir une solution. On sépare la phase organique, on la lave avec une solution saturée de chlorure de sodium et on chasse les solvants sous vide. On reprend le résidu avec 200 ml d'un mélange 1/1 d'acétate d'éthyle et d'acétone, on chauffe au reflux, on laisse refroidir lentement, on sépare les cristaux par filtration et on les sèche. On recueille des cristaux blancs brillants. Point de fusion: 168–170 °C $[\alpha]_D^{20}$=17,6° (c=1,0035, $CHCl_3$).

On concentre la liqueur mère issue de la salification avec la quinidine, on la traite avec de l'acide chlorhydrique à 10% et on extrait le mélange par de l'éther éthylique. On sépare la phase éthérée, on l'évapore et on fait cristalliser le résidu dans 200 ml d'un mélange 1/1 d'acétate d'éthyle et d'acétone. On termine la purification comme pour l'énantiomère dextrogyre. Point de fusion: 168–170 °C $[\alpha]_D^{20}$=18,3° (c=1,002, $CHCl_3$).

Exemple 3. Acide α-méthyl oxo-11 hexahydro-6, 6a, 7, 10, 10a, 11 dibenzo[be]oxépinne-3-acétique.

a) Oxo-5 tétrahydro-2, 3, 4, 5 benzo[b]oxépinne-8-acétate de méthyle. On estérifie 42 g de l'acide obtenu selon l'exemple 2a en le chauffant pendant 2 h au reflux dans 500 ml de méthanol en présence de 5 ml d'acide sulfurique concentré. On évapore le mélange à sec, on reprend le résidu avec un mélange de dichlorométhane et d'une solution aqueuse saturée de carbonate de sodium, on sépare la phase organique, on la lave, on la sèche, on sépare le solide minéral par filtration et on évapore le filtrat. Il reste une huile qu'on distille sous vide.

b) α-Méthyl 0xo-5 tétrahydro-2, 3, 4, 5 benzo-[b]oxépinne-8-acétate de méthyle. On prépare du diisopropylamidure de lithium en mélangeant, dans 100 ml de tétrahydrofuranne, 3,11 ml de diisopropylamine et 17,6 ml de n-butyllithium, à −30 °C. On refroidit le mélange à −70 °C et on y ajoute lentement une solution de 2,34 g de l'ester précédemment préparé dans 20 ml de tétrahydrofuranne; il se forme un précipité jaune-orange, et on agite la suspension pendant 2 h. On ajoute alors 0,74 ml de iodométhane, on agite 30 minutes à −50 °C et on obtient une solution. On laisse revenir à température ambiante, on agite encore 30 minutes, puis on refroidit la solution à 0 °C et on lui ajoute 5 ml de solution saturée de chlorure d'ammonium et 20 ml d'acide chlorhydrique à 5%. On ajoute 200 ml d'eau et on extrait le mélange deux fois avec 200 ml de dichlorométhane. On sépare les phases organiques, on les réunit, on les sèche et on les évapore. Il reste une huile qu'on purifie par chromatographie sur colonne de silice en éluant avec un mélange 3/7 d'acétate d'éthyle/cyclohexanne. On obtient une huile incolore.

c) Acide α-méthyl oxo-11 hexahydro-6, 6a, 7, 10, 10a, 11 dibenzo[be]oxépinne-3-acétique.

On procède dans des conditions analogues à celles décrites dans l'exemple 1f à 1h, en utilisant l'huile précédemment obtenue. Point de fusion: 158–160 °C.

Le tableau suivant illustre les structures et propriétés physiques de quelques composés selon l'invention.

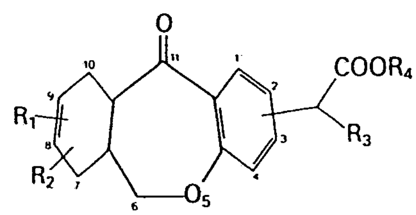

(I)

| Composé | (*) | $R_1$ | $R_2$ | $R_3$ | $R_4$ | F(°C) |
|---|---|---|---|---|---|---|
| 1 (Ex. 1h) | 2 | H | H | H | H | 112–113 |
| 2 (Ex. 1g) | 2 | H | H | H | $CH_3$ | E = 200 °C à 0,15 mBar |
| 3 (**) | 2 | 8–$CH_3$ | H | H | H | 133–134 |
| 4 | 2 | 8–$CH_3$ | 9–$CH_3$ | H | H | 166–167 |
| 5 | 2 | 8–$CH_3$ | 9–$CH_3$ | H | $CH_3$ | 77–78 |
| 6 | 2 | 10–$CH_3$ | H | H | H | 124–125 |
| 7 | 2 | 8–$nC_4H_9$ | 9–$nC_4H_9$ | H | H | huile |
| 8 | 2 | 8–$C_2H_5$ | 9–$C_2H_5$ | H | H | huile |
| 9 | 3 | H | H | H | H | 112–113 |
| 10 | 3 | H | H | H | $CH_3$ | E = 195–200 à 0,25 mBar |
| 11 (Ex. 3) | 3 | H | H | $CH_3$ | H | 158–160 |
| 12 | 3 | 8–$CH_3$ | H | H | H | 158–159 |
| 13 (Ex. 2 b) | 3 | 8–$CH_3$ | 9–$CH_3$ | H | H | 168–170 |
| 14(Ex. 2 b) | 3 | 8–$CH_3$ | 9–$CH_3$ | H | $CH_3$ | 123–125 |
| 15 (Ex. 2 c) | 3 | 8–$CH_3$ | 9–$CH_3$ | H | H | 167–169 $[\alpha]_D^{20}$ = +17,6° |
| 16 (Ex. 2 c) | 3 | 8–$CH_3$ | 9–$CH_3$ | H | H | 167–169 $[\alpha]_D^{20}$ = −18,3° |
| 17 (**) | 3 | 8–$CH_3$ | H | $CH_3$ | H | 131–132 |
| 18 | 3 | 10–$CH_3$ | H | H | H | 95–100 |
| 19 | 3 | 8–$CH_3$ | 9–$CH_3$ | $CH_3$ | H | 139–142 |
| 20 | 3 | 8–$nC_4H_9$ | 9–$nC_4H_9$ | H | H | huile |
| 21 | 3 | 8–$iC_4H_9$ | 9–$iC_4H_9$ | H | H | huile |
| 22 | 3 | 7–$CH_3$ | 10–$CH_3$ | H | H | 164–166 |
| 23 | 3 | 9–$CH_3$ | H | H | H | 149–150 |
| 24 | 3 | 8–$C_2H_5$ | 9–$C_2H_5$ | H | H | huile |
| 25 | 3 | 8–$nC_3H_7$ | 9–$nC_3H_7$ | H | H | huile |
| 26 | 3 | 9–$CH_3$ | 8–$SC_6H_5$ | H | $CH_3$ | huile |
| 27 | 3 | 9–$CH_3$ | 8–$SO_2C_6H_5$ | H | $CH_3$ | 83–84 |
| 28 | 3 | 7–$CH_2$–$CH_2$–10 | | H | H | 131–133 |
| 29 | 3 | 8–CH=CH–CH=CH–9 | | H | H | 176–177 |

(* ): position du groupe –$CH(R_3)COOR_4$
(**): composé contenant une fraction non négligeable d'isomère méthylé en position 9 au lieu de 8.

Les composés de l'invention ont fait l'objet d'essais pharmacologiques qui mettent en évidence leur intérêt comme substances à activités thérapeutiques.

Pour l'étude de l'activité anti-inflammatoire, des essais ont été effectués sur le test de l'œdème à la carragénine chez le rat selon la méthode de Winter et al. («Carrageenin induced edema in hind paw of the rat as an assay for antiinflammatory drugs». Proc. Soc. Exp. Biol. Med. 1962, 111, 544–547). Les animaux utilisés sont des rats mâles Sprague Dawley SPF de Charles River (France) d'un poids moyen de 150 g, répartis en lots au hasard à l'aide d'une table de répartition. Les composés sont administrés par voie orale à des doses comprises entre 1 et 200 mg/kg, 1 heure avant l'injection sous l'aponévrose plantaire à l'une des pattes postérieures, de 0,1 ml de carragénine, en suspension à 1% dans du sérum physiologique stérile. Les animaux témoins ne reçoivent que le placebo, une solution de tween 80 à 1%. L'augmentation du volume de la patte est mesurée 3 heures après l'injection de carragénine au moyen d'un pléthysmomètre Ugo Basile.

Résultats

Par rapport aux animaux témoins, les animaux traités par les composés de l'invention présentent une diminution de 40% du volume de l'œdème à des doses de 2 à 200 mg/kg. Par ailleurs ils ont la propriété inattendue de ne pas être ulcérigènes.

D'autres essais pharmacologiques ont montré que les composés de l'invention sont également actifs comme analgésiques et comme anti-agrégants plaquettaires.

Les résultats montrent que les composés de l'invention peuvent être utilisés comme substances actives de médicaments et compositions pharmaceutiques utilisables pour le traitement d'inflammations d'origines diverses, pour le traitement de la douleur et pour le traitement de l'agrégation des plaquettes. A cet effet ils peuvent être présentés sous toutes formes adaptées à l'administration entérale ou parentérale, par exemple sous forme de comprimés, gélules, dragées, sirops, suppositoires, suspensions buvables ou injectables, en association avec des excipients appropriés. La posologie journalière peut aller de 50 à 1000 mg de substance active.

**Revendications pour les états contractants: BE, CH, DE, FR, GB, IT, LI, LU, NL, SE.**

1. Composés, sous forme de racémates ou d'énantiomères, répondant à la formule générale I

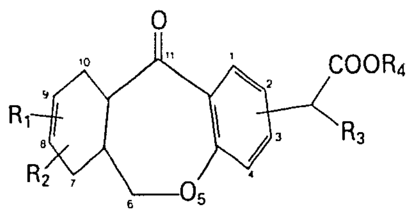

(I)

dans laquelle $R_1$, pris isolément, représente un atome d'hydrogène ou un groupe alkyle en $C_1$–$C_4$, et $R_2$, pris isolément, représente un atome d'hydrogène, un groupe alkyle en $C_1$–$C_4$ ou un groupe phénylthio ou phénylsulfonyle, ou bien $R_1$ et $R_2$ forment ensemble un pont éthano ou, avec les deux atomes de carbone 8 et 9, forment ensemble un noyau benzo condensé, $R_3$ représente un atome d'hydrogène ou un groupe méthyl, et $R_4$ représente un atome d'hydrogène, un groupe alkyle en $C_1$–$C_4$, ou un cation d'une base acceptable en pharmacologie, le groupe $CH(R_3)COOR_4$ étant en position 2 ou 3.

2. Composés selon la revendication 1, caractérisés en ce que $R_3$ et $R_4$ représentent chacun un atome d'hydrogène.

3. Composés selon la revendication 2, caractérisés en ce que $R_1$ et $R_2$ représentent chacun un atome d'hydrogène.

4. Composés selon la revendication 2, caractérisés en ce que $R_1$ et $R_2$ représentent chacun un groupe méthyle.

5. L'acide diméthyl-8,9 oxo-11 hexahydro-6, 6a, 7, 10, 10a, 11 dibenzo[be]oxépinne-3 acétique et ses sels acceptables en pharmacologie, sous formes de racémate ou d'énantiomères.

6. L'isomère d(+) de l'acide diméthyl-8,9 oxo-11 héxahydro-6, 6a, 7, 10, 10a, 11 dibenzo [be]oxépinne-3 acétique, et ses sels acceptables en pharmacologie.

7. Procédé de préparation des composés selon la revendication 1, caractérisé en ce qu'on fait réagir des composés de formules II et III

$R'OCO(CH_2)_3Br$

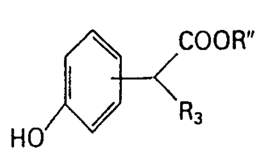

(II) (III)

dans lesquelles R' et R" représentent chacun un groupe alkyle en $C_1$–$C_4$ et $R_3$ est tel que défini dans la revendication 1, puis on hydrolyse le diester obtenu, de formule IV

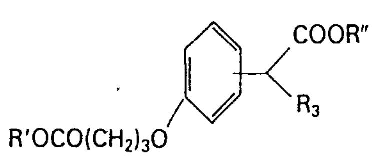

(IV)

en un diacide de formule V

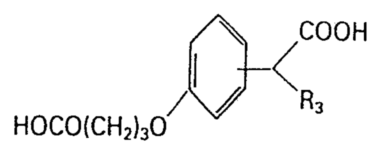

(V)

puis on cyclise ce dernier pour obtenir un composé de formule VI

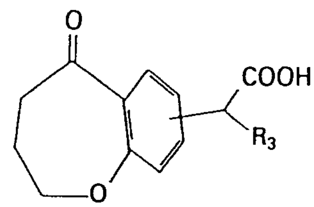

(VI)

que l'on estérifie avec un alcanol R-OH pour obtenir un ester formule VII

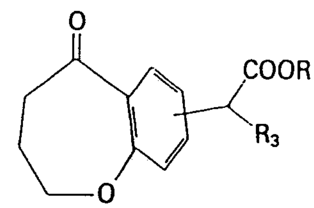

(VII)

dans laquelle R représente un groupe alkyle en $C_1$–$C_4$, puis, en passant par le dérivé 0-triméthylsilylé, on effectue une déshydrogénation donnant un ester de formule VIII

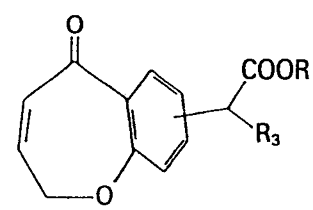

(VIII)

que l'on soumet à une réaction de Diels-Alder avec un butadiène de formule IX

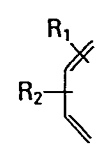

(IX)

dans laquelle $R_1$ et $R_2$ sont tels que définis dans la revendication 1, et, si on le désire, on hydrolyse le composé de formule I ainsi obtenu, dans laquelle $R_4$ est un groupe alkyle en $C_1$–$C_4$, pour obtenir un composé de formule I dans laquelle $R_4$ est un atome d'hydrogène.

8. Procédé selon la revendication 7, caractérisé en ce qu'on part d'un composé de formule III dans laquelle $R_3$ représente un atome d'hydrogène, et on soumet le composé de formule VII à une méthylation en α avant d'effectuer la réaction de Diels-Alder.

9. Médicament, caractérisé en ce qu'il consiste en un composé selon l'une quelconque des revendications 1 à 6.

10. Composition pharmaceutique, caractérisée en ce qu'elle contient un composé selon l'une quelconque des revendications 1 à 6, en association avec un excipient approprié.

**Revendications pour l'état contractant: AT.**

1. Procédé de préparation de composés répondant à la formule générale (I)

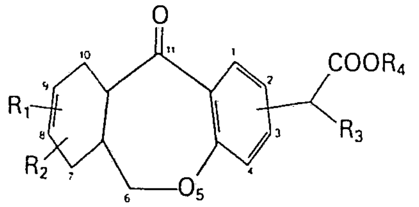

(I)

dans laquelle $R_1$, pris isolément, représente un atome d'hydrogène ou un groupe alkyle en $C_1$–$C_4$, et $R_2$, pris isolément, représente un atome d'hydrogène, un groupe alkyle en $C_1$–$C_4$ ou un groupe phénylthio ou phénylsulfonyle, ou bien $R_1$ et $R_2$ forment ensemble un pont éthano ou, avec les deux atomes de carbone 8 et 9, forment ensemble un noyau benzo condensé, $R_3$ représente un atome d'hydrogène ou un groupe méthyl, et $R_4$ représente un atome d'hydrogène, un groupe alkyle en $C_1$–$C_4$, ou un cation d'une base acceptable en pharmacologie, le groupe $CH(R_3)COOR_4$ étant en position 2 ou 3, procédé caractérisé en ce qu'on fait réagir des composés de formules II et III

$R'OCO(CH_2)_3Br$

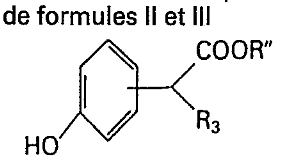

(II) (III)

dans lesquelles R' et R" représentent chacun un groupe alkyle en $C_1$–$C_4$ et $R_4$ est tel que défini ci-dessus, puis on hydrolyse le diester obtenu, de formule IV

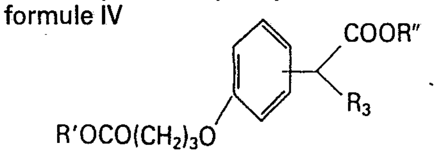

(IV)

en un diacide de formule V

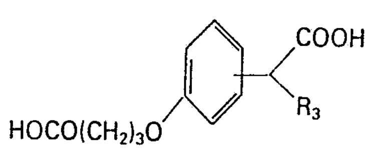

(V)

puis on cyclise ce dernier pour obtenir un composé de formule VI

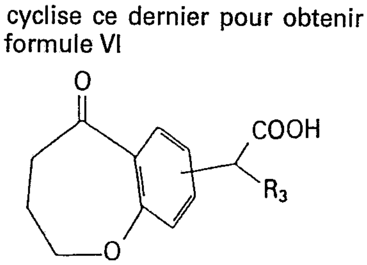

(VI)

que l'on estérifie avec un alcanol R-OH pour obtenir un ester de formule VII

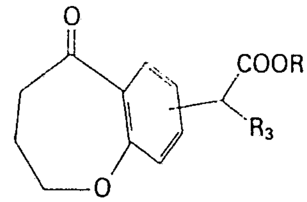

(VII)

dans laquelle R représente un groupe alkyle en $C_1$–$C_4$, puis, en passant par le dérivé 0-triméthylsilylé, on effectue une déshydrogénation donnant un ester de formule VIII

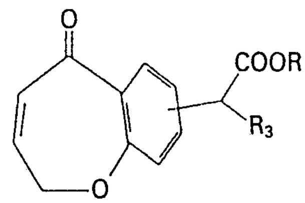

(VIII)

que l'on soumet à une réaction de Diels-Alder avec un butadiène de formule IX

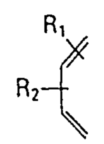

(IX)

dans laquelle $R_1$ et $R_2$ sont tels que définis ci-dessus, et, si on le désire, on hydrolyse le composé de formule I ainsi obtenu, dans laquelle $R_4$ est un groupe alkyle en $C_1$–$C_4$, pour obtenir un composé de formule I dans laquelle $R_4$ est un atome d'hydrogène.

2. Procédé selon la revendication 1, caractérisé en ce qu'on part d'un composé de formule III dans laquelle $R_3$ représente un atome d'hydrogène, et on soumet le composé de formule VII à une méthylation en α avant d'effectuer la réaction de Diels-Alder.

**Patentansprüche für die Vertragsstaaten BE, CH, DE, FR, GB, IT, LI, LU, NL, SE**

1. Verbindungen in Form der Racemate oder Enantiomeren entsprechend der allgemeinen Formel I

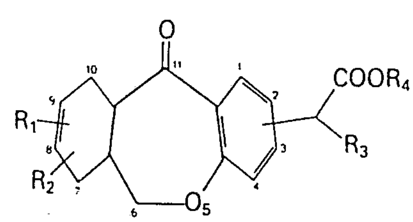

(I)

worin $R_1$ für sich allein ein Wasserstoffatom oder eine $C_1$–$C_4$-Alkylgruppe und $R_2$ für sich allein ein Wasserstoffatom eine $C_1$–$C_4$-Alkylgruppe oder eine Phenylthio- oder Phenylsulfonylgruppe bedeutet oder aber $R_1$ und $R_2$ gemeinsam eine Ethanobrücke darstellen oder zusammen mit den beiden Kohlenstoffatomen 8 und 9 einen kondensierten

Benzolring bedeuten, $R_3$ für ein Wasserstoffatom oder eine Methylgruppe und $R_4$ für ein Wasserstoffatom, eine $C_1$–$C_4$-Alkylgruppe oder ein Kation einer in der Pharmakologie verwendbaren Base steht, wobei die Gruppe $CH(R_3)COOR_4$ in der 2- oder 3- Stellung steht.

2. Verbindungen nach Anspruch 1, dadurch gekennzeichnet, dass $R_3$ und $R_4$ jeweils für ein Wasserstoffatom stehen.

3. Verbindungen nach Anspruch 2, dadurch gekennzeichnet, dass $R_1$ und $R_2$ jeweils für ein Wasserstoffatom stehen.

4. Verbindungen nach Anspruch 2, dadurch gekennzeichnet, dass $R_1$ und $R_2$ jeweils für eine Methylgruppe stehen.

5. 8,9-Dimethyl-11-oxo-6, 6a, 7, 10, 10a, 11-hexahydro-dibenzo[be]-oxepin-3-essigsäure und ihre pharmakologisch verwendbaren Salze, in Form des Recemats oder der Enantiomeren.

6. Das d(+)-Isomere der 8,9-Dimethyl-11-oxo-6, 6a, 7, 10, 10a, 11-hexahydro-dibenzo[be]-oxepin-3-essigsäure und deren pharmakologisch verwendbare Salze.

7. Verfahren zur Herstellung der Verbindungen nach Anspruch 1, dadurch gekennzeichnet, dass man Verbindungen der Formeln II und III

$R'OCO(CH_2)_3Br$

(II)

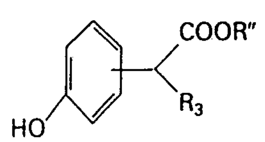

(III)

in welchen R' und R" jeweils für eine $C_1$–$C_4$-Alkylgruppe stehen und $R_3$ die in Anspruch 1 genannte Bedeutung hat, reagieren lässt, dann den erhaltenen Diester der Formel IV

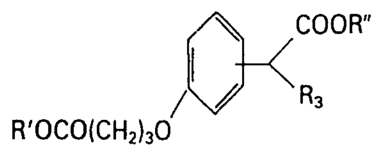

(IV)

zu einer Disäure der Formel V

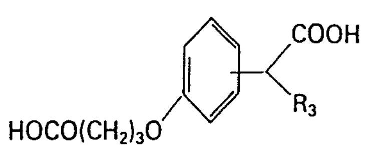

(V)

hydrolysiert, anschliessend diese letztere zur Gewinnung einer Verbindung der Formel VI

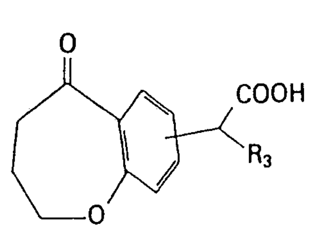

(VI)

cyklisiert, worauf man diese mit einem Alkohol R-OH verestert zur Herstellung eines Esters der Formel VII

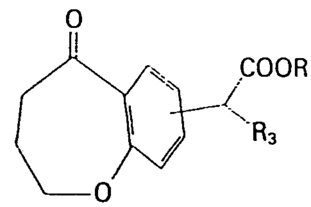

(VII)

in welcher R für eine $C_1$–$C_4$-Alkylgruppe steht, worauf man über das 0-Trimethylsilyl-Derivat eine Dehydrierung vornimmt, die einen Ester der Formel VIII

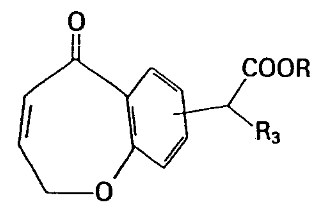

(VIII)

ergibt, den man einer Diels-Alder-Reaktion mit einem Butadien der Formel IX

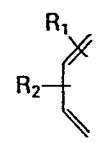

(IX)

in welcher $R_1$ und $R_2$ die in Anspruch 1 genannte Bedeutung haben, unterwirft und gewünschtenfalls die so erhaltene Verbindung der Formel I, in welcher $R_4$ eine $C_1$–$C_4$-Alkylgruppe darstellt, zur Gewinnung einer Verbindung der Formel I, in welcher $R_4$ ein Wasserstoffatom bedeutet, hydrolysiert.

**8. Verfahren nach Anspruch 7, dadurch gekennzeichnet, dass man von einer Verbindung der Formel III ausgeht, in welcher $R_3$ für ein Wasserstoffatom steht, und dass man die Verbindung der Formel VII vor der Durchführung der Diels-Alder-Reaktion einer Methylierung in α unterwirft.**

**9. Arzneimittel, dadurch gekennzeichnet, dass es aus einer Verbindung nach einem der Ansprüche 1 bis 6 besteht.**

**10. Pharmazeutische Zusammensetzung, dadurch gekennzeichnet, dass sie eine Verbindung nach einem der Ansprüche 1 bis 6 in Kombination mit einem geeigneten Bindemittel enthält.**

**Patentansprüche für den Vertragsstaat AT**

**1. Verfahren zur Herstellung von Verbindungen der allgemeinen Formel (I)**

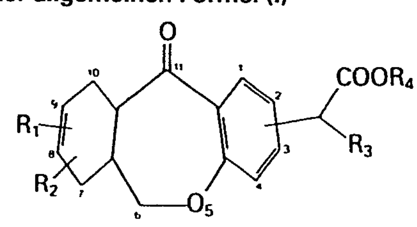

(I)

worin $R_1$ für sich allein ein Wasserstoffatom oder eine $C_1$–$C_4$-Alkylgruppe und $R_2$ für sich allein ein

Wasserstoffatom, eine $C_1$–$C_4$-Alkylgruppe oder eine Phenylthio- oder Phenylsulfonylgruppe bedeutet oder aber $R_1$ und $R_2$ gemeinsam eine Ethanobrücke darstellen oder zusammen mit den beiden Kohlenstoffatomen 8 und 9 einen kondensierten Benzolring bedeuten, $R_3$ für ein Wasserstoffatom oder eine Methylgruppe und $R_4$ für ein Wasserstoffatom, eine $C_1$–$C_4$-Alkylgruppe oder ein Kation einer in der Pharmokologie verwendbaren Base steht, wobei die Gruppe $CH(R_3)COOR_4$ in der 2- oder 3- Stellung steht, dadurch gekennzeichnet, dass man die Verbindungen der Formeln II und III

$R'OCO(CH_2)_3Br$

(II)

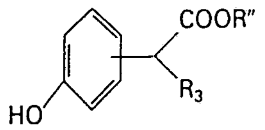

(III)

in welchen R' und R" jeweils für eine $C_1$–$C_4$-Alkylgruppe stehen und $R_3$ die oben genannte Bedeutung hat, reagieren lässt, dann den erhaltenen Diester der Formel IV

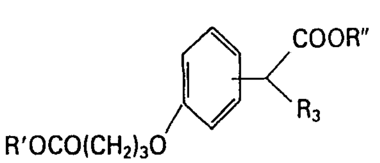

(IV)

zu einer Disäure der Formel V

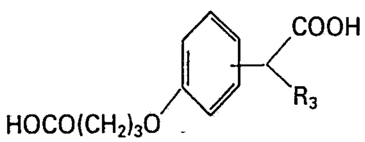

(V)

hydrolysiert, anschliessend diese letztere zur Gewinnung einer Verbindung der Formel VI

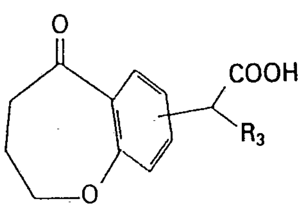

(VI)

cyklisiert, worauf man diese mit einem Alkohol R-OH verestert zur Herstellung eines Esters der Formel VII

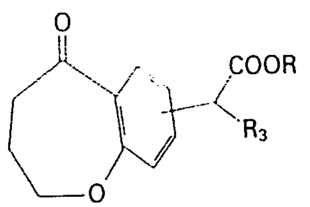

(VII)

in welcher R für eine $C_1$–$C_4$-Alkylgruppe steht, worauf man über des O-Trimethylsilyl-Derivat eine Dehydrierung vornimmt, die einen Ester der Formel VIII

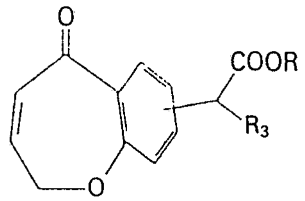

(VIII)

ergibt, den man einer Diels-Alder-Reaktion mit einem Butadien der Formel IX

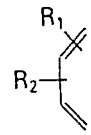

(IX)

in welcher $R_1$ und $R_2$ die oben genannte Bedeutung haben, unterwirft und gewünschtenfalls die so erhaltene Verbindung der Formel I, in welcher $R_4$ eine $C_1$–$C_4$-Alkylgruppe darstellt, zur Gewinnung einer Verbindung der Formel I, in welcher $R_4$ ein Wasserstoffatom bedeutet, hydrolysiert.

2. Verfahren nach Anspruch 1, dadurch gekennzeichnet, dass man von einer Verbindung der Formel III ausgeht, in welcher $R_3$ für ein Wasserstoffatom steht, und dass man die Verbindung der Formel VII vor der Durchführung der Diels-Alder-Reaktion einer Methylierung in α unterwirft.

**Claims for the contracting states: BE, CH, DE, FR, GB, IT, LI, LU, NL, SE**

1. Compounds in the form of racemates or enantiomers, corresponding to the general formula I

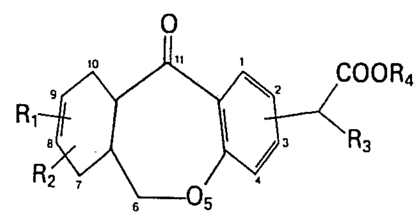

(I)

in which $R_1$, taken separately, denotes a hydrogen atom or a $C_1$–$C_4$ alkyl group, and $R_2$, taken separately, denotes a hydrogen atom, a $C_1$–$C_4$ alkyl group or a phenylthio or phenylsulphonyl group, or alternatively $R_1$ and $R_2$ together form an ethano bridge or, together with the two carbon atoms 8 and 9, form a fused benzene ring, $R_3$ denotes a hydrogen atom or a methyl group and $R_4$ denotes a hydrogen atom, a $C_1$–$C_4$ alkyl group or a cation of a base which is acceptable in pharmacology, the group $CH(R_3)COOR_4$ being at position 2 or 3.

2. Compounds according to Claim 1, characterized in that $R_3$ and $R_4$ each denote a hydrogen atom.

3. Compounds according to Claim 2, characterized in that $R_1$ and $R_2$ each denote a hydrogen atom.

4. Compounds according to Claim 2, characterized in that $R_1$ and $R_2$ each denote a methyl group.

5. 8,9-Dimethyl-11-oxo-6,6a,7,10,10a,11-hexahydro-dibenz[be]oxepin-3-acetic acid and its salts which are acceptable in pharmacology, in the forms of racemate or enantiomers.

6. The d(+) isomer of 8,9-dimethyl-11-oxo-6, 6a, 7, 10, 10a, 11-hexahydro-dibenz[be]oxepin-3-acetic acid, and its salts which are acceptable in pharmacology.

7. Process for preparing the compounds according to Claim 1, characterized in that compounds of formulae II and III

$R'OCO(CH_2)_3Br$

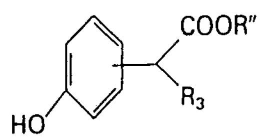

(II) (III)

in which R' and R" each denote a $C_1$–$C_4$ alkyl group and $R_3$ is as defined in Claim 1, are reacted, the diester obtained, of formula IV

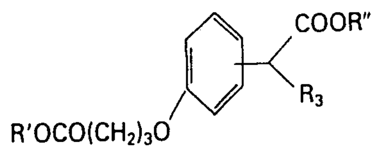

(IV)

is then hydrolysed to a diacid of formula V

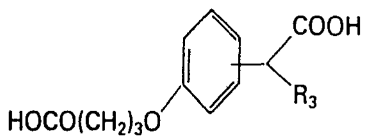

(V)

and the latter is then cyclized to obtain a compound of formula VI

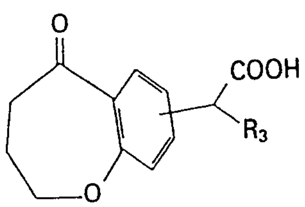

(VI)

which is esterified with an alkanol R-OH to obtain an ester of formula VII

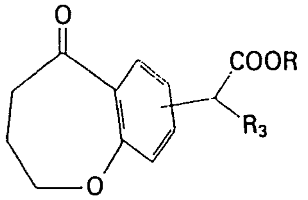

(VII)

in which R denotes a $C_1$–$C_4$ alkyl group, then, passing through the 0-trimethylsilyl derivative, a dehydrogenation is performed to give an ester of formula VIII

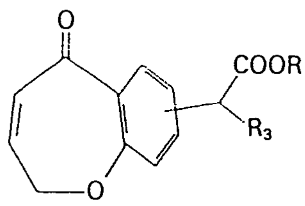

(VIII)

which is subjected to a Diels-Alder reaction with a butadiene of formula IX

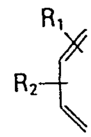

(IX)

in which $R_1$ and $R_2$ are as defined in Claim 1, and, if so desired, the compound of formula I thereby obtained, in which $R_4$ is a $C_1$–$C_4$ alkyl group, is hydrolysed to obtain a compound of formula I in which $R_4$ is a hydrogen atom.

8. Process according to Claim 7, characterized in that the starting substance is a compound of formula III in which $R_3$ denotes a hydrogen atom, and the compound of formula VII is subjected to an α-methylation before performing the Diels-Alder reaction.

9. Drug, characterized in that it consists of a compound according to any one of Claims 1 to 6.

10. Pharmaceutical composition, characterized in that it contains a compound according to any one of Claims 1 to 6, in combination with a suitable exipient.

**Claims for the contracting state: AT**

1. Process for preparing compounds corresponding to the general formula (I)

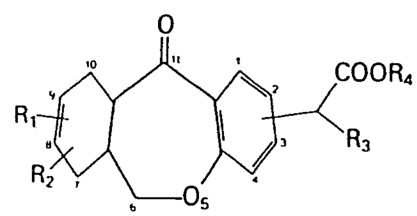

(I)

in which $R_1$, taken separately, denotes a hydrogen atom or a $C_1$–$C_4$ alkyl group, and $R_2$, taken separately, denotes a hydrogen atom, a $C_1$–$C_4$ alkyl group or a phenylthio or phenylsulphonyl group, or alternatively $R_1$ and $R_2$ together form an ethano bridge or, together with the two carbon atoms 8 and 9, form a fused benzene ring, $R_3$ denotes a hydrogen atom or a methyl group and $R_4$ denotes a hydrogen atom, a $C_1$–$C_4$ group or a cation of a base which is acceptable in pharmacology, the group $CH(R_3)COOR_4$ being at position 2 or 3,

which process is characterized in that compounds of formulae II and III

$R'OCO(CH_2)_3Br$

(II)

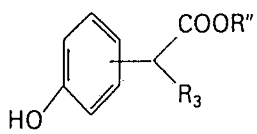

(III)

in which R' and R" each denote a $C_1$–$C_4$ alkyl group and $R_3$ is as defined above, are reacted, the diester obtained, of formula IV

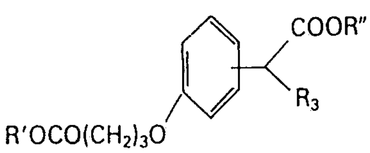

(IV)

is then hydrolysed to a diacid of formula V

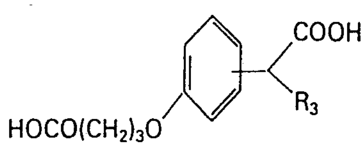

(V)

and the latter is then cyclized to obtain a compound of formula VI

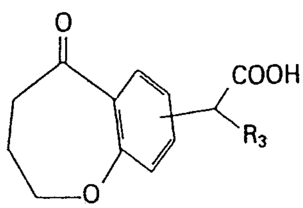

(VI)

which is esterified with an alkanol R-OH to obtain an ester of formula VII

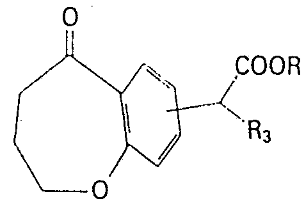

(VII)

in which R denotes a $C_1$–$C_4$ alkyl group, then, passing through the 0-trimethylsilyl derivative, a dehydrogenation is performed to give an ester of formula VIII

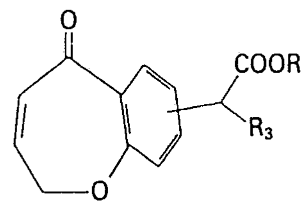

(VIII)

which is subjected to a Diels-Alder reaction with a butadiene of formula IX

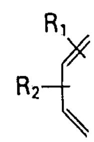

(IX)

in which $R_1$ and $R_2$ are as defined above, and, if so desired, the compound of formula I thereby obtained, in which $R_4$ is a $C_1$–$C_4$ alkyl group, is hydrolysed to obtain a compound of formula I in which $R_4$ is a hydrogen atom.

2. Process according to Claim 1, characterized in that the starting substance is a compound of formula III in which $R_3$ denotes a hydrogen atom, and the compound of formula VII is subjected to an α-methylation before performing the Diels-Alder reaction.